# EUROPEAN PATENT SPECIFICATION

(11) **EP 2 978 389 B1**
(45) Date of publication and mention of the grant of the patent: **17.05.2017**
(21) Application number: 14722347.3
(22) Date of filing: 27.03.2014
(51) Int. Cl.: A61F 2/58, B29C 39/14, A61F 2/50, A61F 2/76

(54) **METHOD AND DEVICE FOR MAKING FINGERPRINTS ASSOCIATED TO ARTIFICIAL FINGERS**
VERFAHREN UND VORRICHTUNG ZUR HERSTELLUNG VON FINGERABDRÜCKEN AUF KÜNSTLICHEN FINGERN
PROCÉDÉ ET DISPOSITIF POUR FABRIQUER DES EMPREINTES DIGITALES ASSOCIÉES À DES DOIGTS ARTIFICIELS

(30) Priority: 29.03.2013 IT PI20130023
(43) Date of publication of application: 03.02.2016
(73) Proprietor: Scuola Superiore di Studi Universitari e di Perfezionamento Sant'Anna, 56125 Pisa (IT)
(72) Inventor: MUSCOLO, Giovanni Gerardo, I-56025 Pontedera ( PI) (IT); ODDO, Calogero Maria, I-56025 Pontedera ( PI) (IT); CARROZZA, Maria Chiara, I-56025 Pontedera (PI) (IT); BECCAI, Lucia, I-56025 Pontedera (PI) (IT)
(74) Representative: Leotta, Antonio
(86) International application number: PCT/IB2014/060223
(87) International publication number: WO 2014/155338

(56) References cited:
- US-B1- 6 177 034
- KOH HOSODA ET AL: "Anthropomorphic robotic soft fingertip with randomly distributed receptors", ROBOTICS AND AUTONOMOUS SYSTEMS, vol. 54, 2006, pages 104-109, XP002721452,
- yaroslav tenzer et al: Inexpensive and Easily Customized Tactile Array Sensors using MEMS Barometers Chips , 2012, pages 1-5, XP002721453, Retrieved from the Internet: URL:http://biorobotics.harvard.edu/pubs/20 12/journal/2012_YTenzer_BarometricSensors. pdf [retrieved on 2014-03-11]
- CALOGERO M. ODDO ET AL: "A Biomometic MEMS-based Tactile Sensor array with Fingerprints integrated in a Robotic Fingertip for Artificial Roughness Encoding", PROCEEDINGS OF THE 2009 IEEE, INTERNATIONAL CONFERENCE ON ROBOTICS AND BIOMIMETICS, 19 December 2009 (2009-12-19), - 23 December 2009 (2009-12-23), pages 894-900, XP002721454, cited in the application

## Description

### Technical Field

The present invention relates to a method for producing silicone coatings on sensorized items and, more particularly, aimed at producing an artificial fingerprint on an artificial finger having an array of tactile sensors.

The invention relates also to a forming device for the production of the above mentioned artificial fingerprints on sensorized items.

The invention has been developed within the research projects mentioned in the following: - European Project EU-FP6-NMP-033287 NANOBIOTACT (Nanoengineering Biomimetic Tactile Sensors); - European Project EU-FP7-NMP-228844 NANOBIOTOUCH (Nano-resolved multi-scale investigations of human tactile sensations and tissue engineered nanobiosensor); - European Project EU-FP7-ICT-611687 NEBIAS (NEurocontrolled BIdirectional Artificial upper limb and hand prosthesis); - Italian Project PRIN/HandBot (Biomechatronic hand prostheses endowed with bio-inspired tactile perception, bi-directional neural interfaces and distributed sensori-motor control; CUP: B81J12002680008; prot.: 20102YF2RY).

### Background Art

In the biomimetic robotics field, artificial fingers have been studied and developed, which are provided with MEMS-based tactile sensor arrays, aimed at detecting the surfaces roughness. The tactile sensors, mounted on PCBs (printed circuit boards) are associated with a fingertip of an artificial finger and then covered with a silicone coating, a kind of artificial skin, which consequently constitutes an interface between the array of sensors and the surface, whose roughness is to be detected.

Methods are known for applying a silicone coating to a sensorized item, such as, for example, the one described in the International Patent Application WO 2012031703, wherein liquid silicone is poured into a die of suitable dimensions, to which a flat item is then coupled. One or more optoelectronic sensors are associated with one surface of the flat item. Afterwards, the silicone is made to solidify by making it adhere to the item and to the sensors, and finally the item is removed from the die. While being extremely simple, the above mentioned method presents application limits and is not particularly suitable for being used for the coating of artificial fingers.

Moreover, the scientific publication 'A Biomimetic MEMS-based Tactile Sensor Array with Fingerprints integrated in a Robotic Fingertip for Artificial Roughness Encoding' published in 'Proceedings of the 2009 IEEE International Conference on Robotics and Biomimetics' of December 2009 points out and documents the advantages deriving from a silicone coating capable of reproducing an artificial fingerprint on the sensorized artificial finger and other advantages deriving from the presence of a coating made of two layers, one of which is internal and smooth and less hard with respect to the other, external and harder, which has ribs arranged in such a way as to reproduce a human fingerprint pattern. KOH HOSODA ET AL: "Anthropomorphic robotic soft fingertip with randomly distributed receptors", ROBOTICS AND AUTONOMOUS SYSTEMS, vol. 54, 2006, pages 104-109, discloses a method for applying a plastic coating to a sensorized item and a respective casting device, comprising the steps of casting a silicone material in a non solid state into a shell and solidification of said silicone material in order to obtain a fingertip of an artificial finger.

The known methods, included the one of the above mentioned Application WO 2012031703, are not able to produce coatings which are appropriate to reproduce a human fingerprint with the above outlined characteristics.

### Disclosure of Invention

### Technical Problem

Therefore, the object of the present invention is to propose a method and a device for the application of a plastic coating on a rigid sensorized item.

Another object of the present invention is to propose a method and device for producing, in a simple and cheap way, a plastic coating, in particular a silicon coating, made by two overlaying layers, on a rigid sensorized item.

Another specific object of the present invention is to propose a method and a device for producing a silicone coating comprising an artificial fingerprint on a fingertip of an artificial finger having an array of tactile sensors.

### Technical Solution

According to an aspect of the present invention, the above and other objects are obtained by a method for application of a plastic coating on a sensorized itemo, comprising the steps of:
- a) - assembling the removable positioning members with the sensorized item,
- b) - blocking the group made of the sensorized itemo and the positioning members in a multi-part casting shell,
- c) - assembling a die with the above mentioned casting shell,
- d) - casting a silicone material in a non solid state into the casting shell,
- e) - solidification of the silicone material,
- f) - disassembling the die and shell,
- g) - removing any casting residues (burrs, sprues, vent channels),
wherein the sensorized item, casting shell and casting die, in a casting configuration, form a casting chamber, a first wall of which includes a surface having an array of sensors of the casting item, and a second wall of which, substantially opposite to the first one, includes a shaped surface with recessed and/or relief pattern of the casting die.

The above outlined method allows a silicone coating, whose outer surface has recessed and/or relief pattern, to be obtained on a sensorized item in a simple way, with great accuracy and repeatability.

Advantageously, steps b) to g) are performed at least twice with different dies, which are aimed at defining, thanks to a different sizing, different heights of the casting chamber, so that at least two superimposed layers of said silicone coating are obtained.

The above outlined method allows to obtain two overlaying coating layers in an extremely simple way, using the same casting shell and positioning members, and simply repeating the steps of casting silicone with two different dies, one of which is dimensioned and shaped to obtain the inner layer and the other to obtain the outer layer.

Also advantageously, the sensorized item is a fingertip of an artificial finger which is provided with MEMS-based tactile sensor array, and a casting die for realizing an outer layer of the coating is shaped in such a way that the above mentioned pattern reproduces a fingerprint.

According to another aspect of the present invention, the above objects are obtained by a casting device for the application of a plastic coating on a sensorized item, comprising:
- a multi-part casting shell,
- positioning members, removably associated with the sensorized item and designed to guide the positioning of the sensorized item in the casting shell, and
- at least one casting die,
wherein the sensorized item, casting shell and casting die, in an assembled casting configuration, form a casting chamber, a first wall of which includes a surface having an array of sensors of the casting item, and a second wall of which, substantially opposite to the first one, includes a shaped surface with recessed and/or relief pattern of the casting die.

Advantageously, the device includes a plurality of the above mentioned casting dies, which can be assembled with the same casting shell, and each of them is dimensioned and shaped in such a way as to form a casting chamber aimed at shaping a specific layer of a respective plurality of layers that form the plastic coating.

A device as outlined above allows to obtain coatings in silicone material having more layers, in which one or more inner layers are softer and have a substantially smooth surface, while the outer layer is harder and has a surface shaped with a recessed and relief pattern, which reproduces the human fingerprint pattern.

### Description of Drawings

These and other characteristics of the invention will be easier to understand from the following description of a preferred embodiment thereof, provided as a non limiting example, with reference to the enclosed figures, in which:
- Figure 1 is a perspective view of a fingertip of an artificial finger;
- Figure 2 is a perspective view of the fingertip of Fig. 1 with a removable positioning element assembled therewith;
- Figure 3 is a perspective view of the fingertip of Fig. 1 with an array of tactile sensors applied thereto;
- Figure 4 shows the fingertip of Fig. 3 with a coating made thereon reproducing a human fingerprint;
- Figure 5 is a section view of the packaging of sensors of Fig. 4;
- Figure 6 is a perspective top view of a casting device of the present invention in the assembled casting configuration;
- Figure 7 shows the device of Fig. 6 in a perspective view from the bottom;
- Figure 8 is an exploded view of the casting device of Fig. 6;
- Figures 9 to 12 show the main components of the casting shell of the device of Fig. 6;
- Figure 13 shows a casting die included in the device of the invention;
- Figure 14 shows another casting die included in the device of the invention.

### Mode for Invention

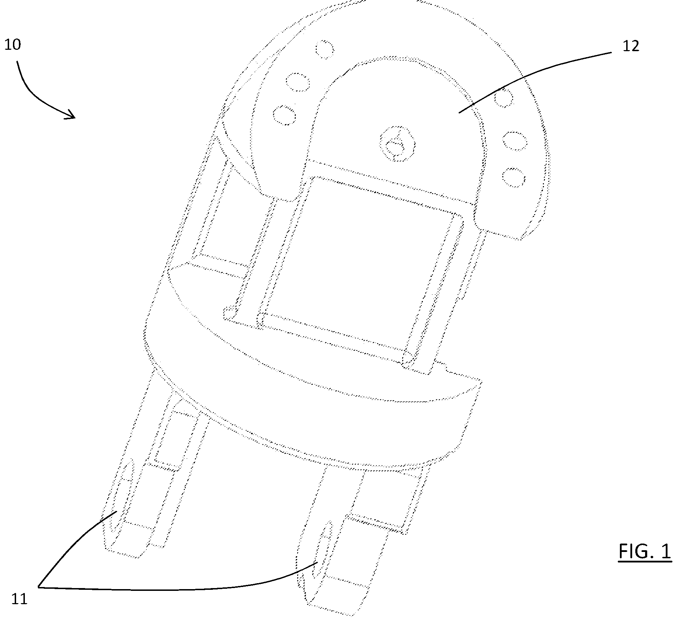

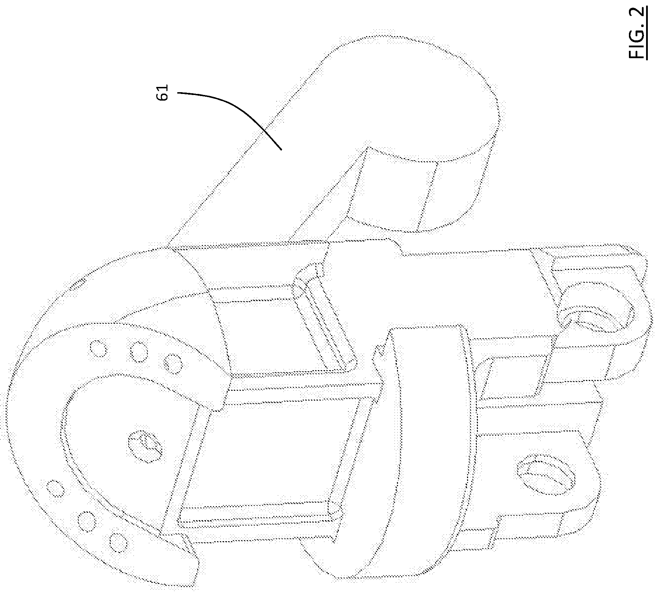

In Figure 1, reference numeral 10 indicates a item, as a whole, that constitutes the fingertip of an artificial finger. The above mentioned fingertip is made of an elongated item structure, which includes hinge coupling means, 11, at one end, for coupling to a respective intermediate phalange of the artificial finger, and, at the opposite end, a surface, 12, which can be provided with sensors and is inclined and turned frontwise. In the rear area of the item 10, there are fastening means of a positioning element, 61, shown in Fig. 2. The positioning element 61 is removable and is aimed at handling the item 10 and indicating its position within the device of the invention.

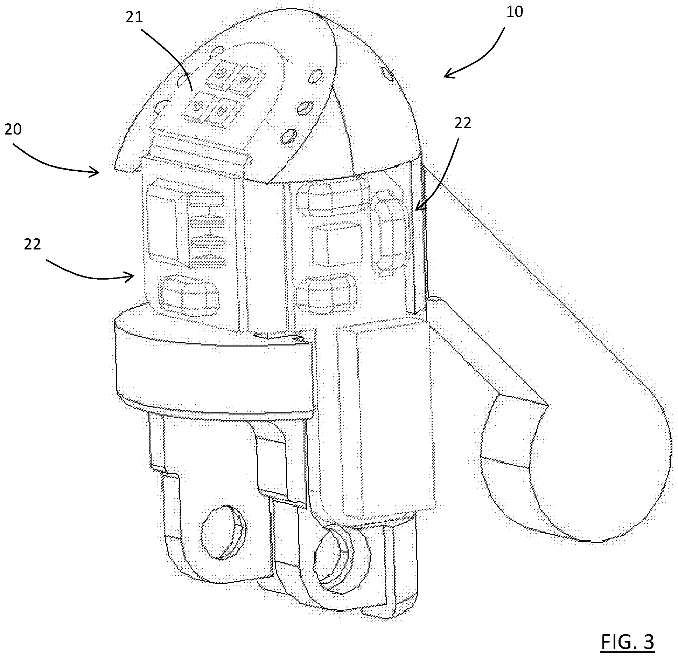

Figure 3 shows a sensorized item composed by the item 10, with a tactile sensor, 20, assembled therewith, which consists of an array of MEMS-based sensors, 21, and the relative control and power electronics, 22, made on printed circuit boards (PCB).

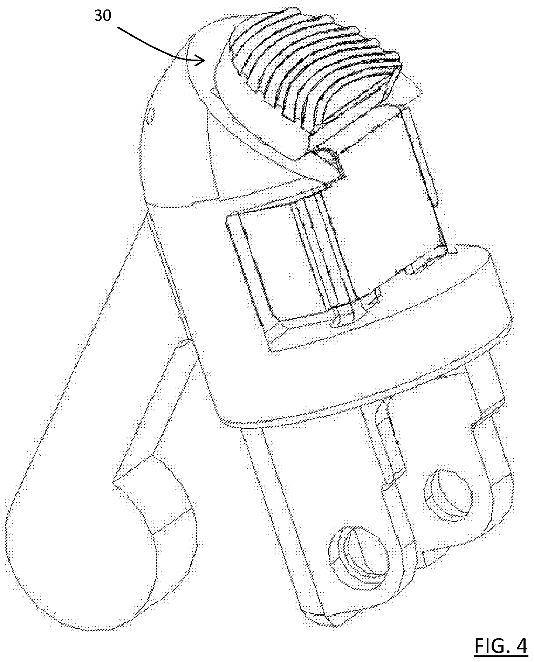

Figure 4 shows the sensorized artificial finger with a silicone coating, 30, made in the area of the array of sensors 21 by using the method and device of the invention. The silicone coating wraps the sensorized surface of the item 10.

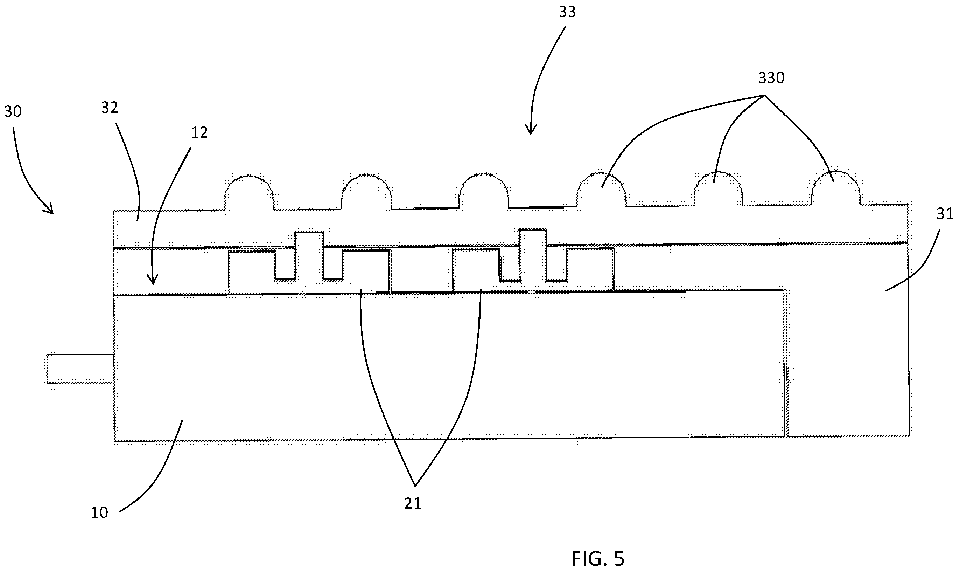

Figure 5 shows, in a section view, the area of the item 10 provided with the coating 30. As it can be seen, the coating 30 consists of two layers: a first, inner coating layer, 31, made of a first silicone material, which coats and wraps the surface 12, on which the sensors are positioned; and a second, outer coating layer, 32, made of a second silicone material, harder with respect to the first one, whose outer surface is provided with ribs, 330, forming, on the outer surface, a recessed and/or relief pattern, 33, which in this embodiment reproduces a human fingerprint pattern.

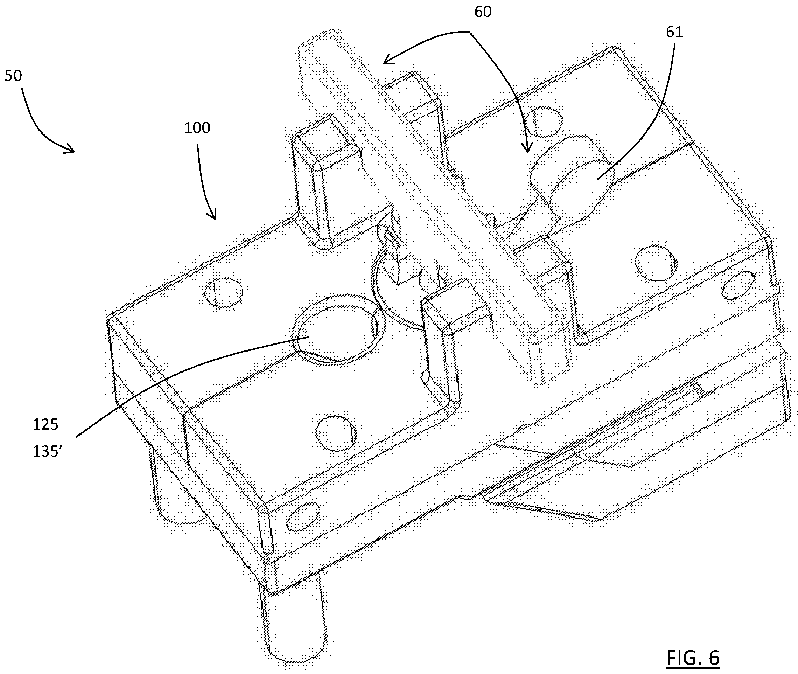

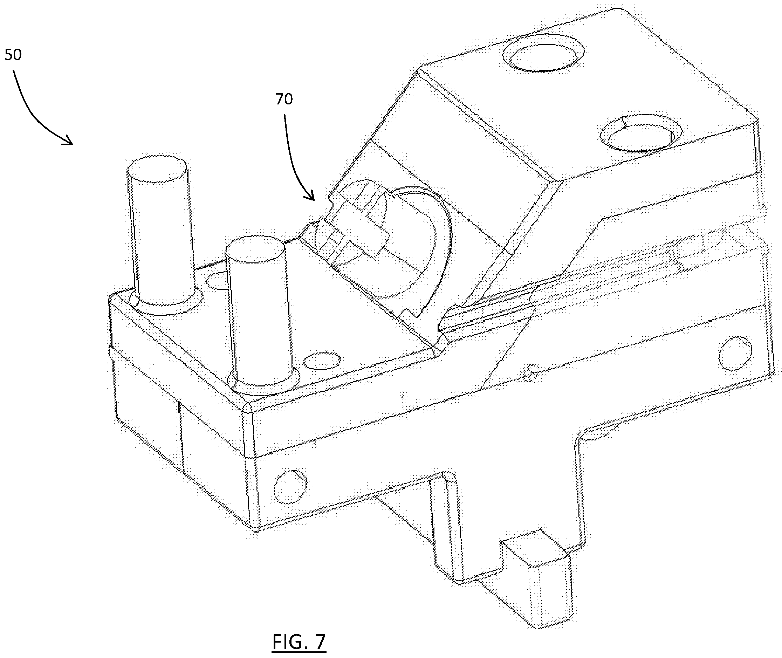

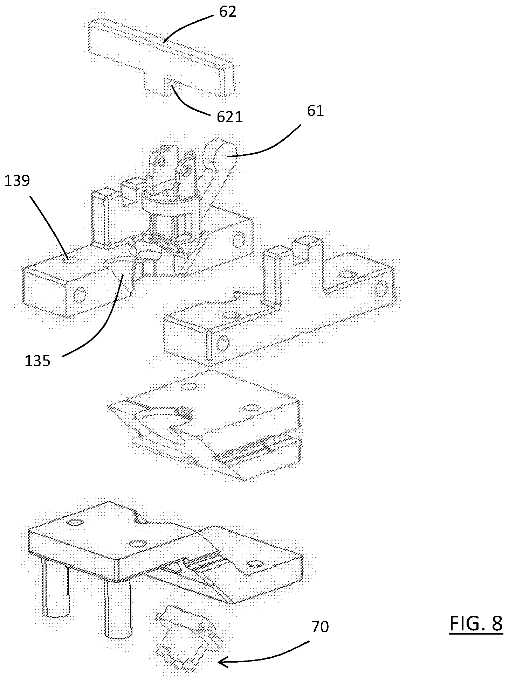

With reference to Figures 6 to 10, reference numeral 50 indicates a casting device as a whole, made according to the present invention, by means of which a silicone coating as described above can be applied to a sensorized artificial finger. The casting device 50 includes substantially a multi-part casting shell, 100, positioning members, 60, aimed at indicating and fastening the item 10 within the casting shell, and at least one casting die, 70, comprising at least one surface shaped in such a way as to obtain the above mentioned recessed and/or relief pattern 33 over the coating 30.

The casting shell 100 consists of four separated main components, which can be assembled to form the casting configuration shown in Figs. 6-8.

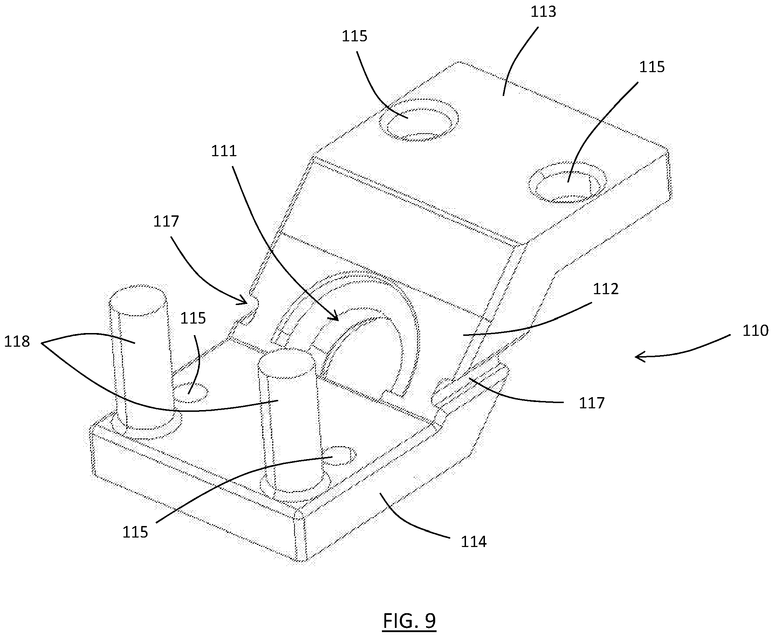

A first component, shown in Fig. 9, includes a lower element, 110, having an opening, 111, aimed at housing the casting die 70 and provided on a portion, 112, which is inclined with respect to two terminal portions 113, 114. The terminal portions feature through holes 115, aimed at housing fastening elements, screw-like or another type of elements, aimed at reciprocally fastening the various components of the multi-part casting shell 100. Grooves, 117, are situated at both sides of the inclined portion 112 to form housings for fastening means of the die 70. The terminal portion 114 is also provided with feet, 118, which, together with the lower surface of the other terminal portion 113, are aimed at creating a resting surface.

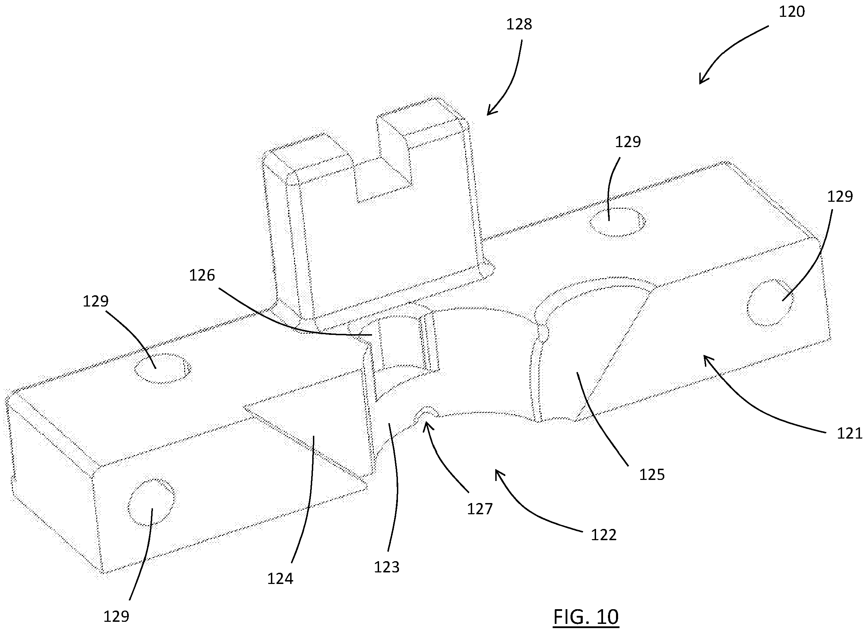

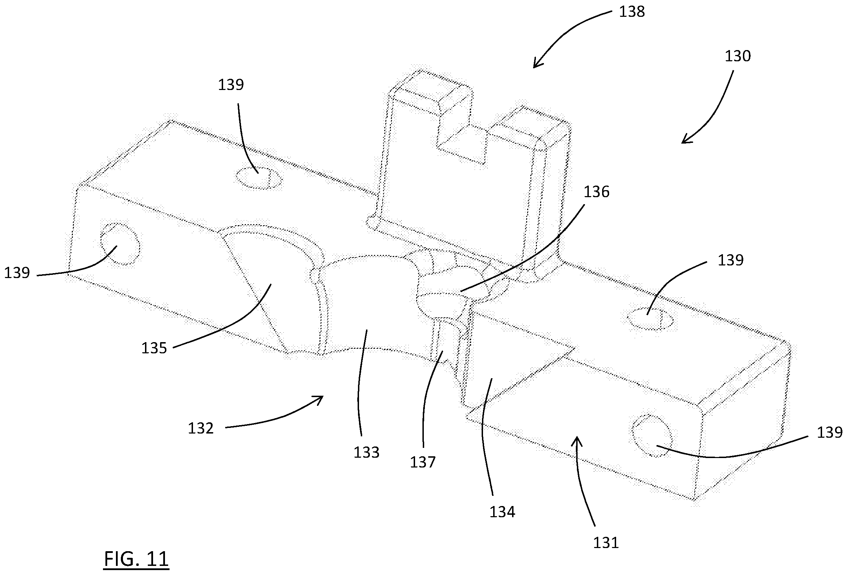

Further components of the casting shell 100, shown in Figs. 10 and 11, include a first and second lateral elements, 120, 130, which are complementary with each other. The above mentioned lateral elements 120 and 130 feature, in correspondence to their coupling surfaces, 121, 131, complementary casting impressions, 122, 132, which, in the assembled casting configuration, form a main housing, 123, 133 for the item 10, an auxiliary housing, 124, 134, for the positioning element 61, sprues, 125, 135, and cavities, 126, 136, for feeding material during the withdrawal step. Further grooves, 127, 137, set the casting impression 122, 132 in communication with the outside and serve as vent channels. The two lateral elements 120 and 130 are also provided with a shaped protuberance, 128, 138, which forms fastening means for a second positioning element, 62, of the positioning members 60. The above mentioned second positioning element 62 consists of an elongated prismatic body, whose shape allows it to be inserted into the above mentioned shaped protuberances 128 and 138, and features a hole 621, situated in an intermediate position. The hole 621 can be engaged with a pin that engages at the same time also the hinge coupling means 11 of the item 10, thus fastening the same item to the second positioning element 62. The lateral elements 120 and 130 feature also through holes, 129, 139 aimed at housing the fastening elements.

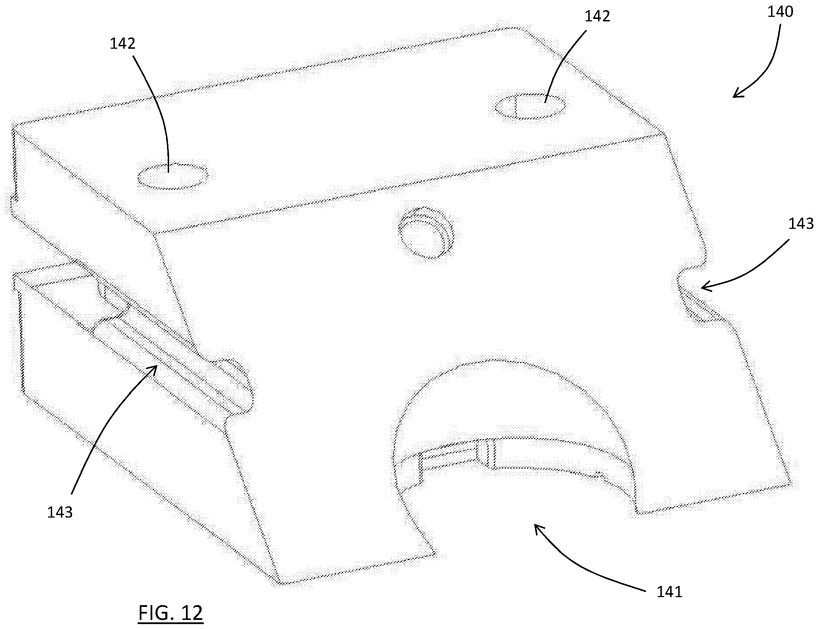

Another component of the casting shell 100 is a rear element, 140, shown in Fig. 12, which is substantially wedge-shaped and includes, an auxiliary impression, 141, in correspondence to a point of said wedge. The impression 141 houses the gates of the item 10 and the relative first positioning element 61. The rear element 140 comprises also holes, 142, for the fastening means and external grooves, 143, which, in the assembled casting configuration, form housings for fastening means of the die 70, together with the grooves 117 of the lower element.

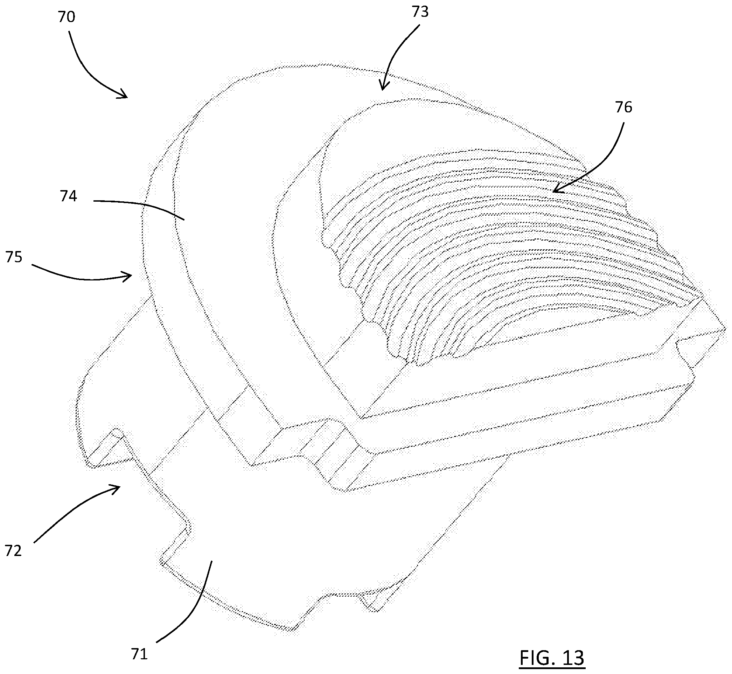

With reference to Figure 13, a die 70 of the casting device 50 features a tail portion, 71, provided with grooves, 72, aimed at housing the means for fastening the die 70 to the casting shell 100, and a head portion, 73, having a shape corresponding to that of the opening 111 made in the lower element 110 up to an abutment surface, 74, of an abutment portion, 75 intermediate between the head portion and the tail portion. The height of the head portion 73 determines the height of a casting chamber and, consequently, the thickness of the layer of coating 30 which will be produced by the device. A casting surface, 76, of the head portion 71 determines the surface shape of the coating 30. In the embodiment of Fig. 13, the casting surface 76 presents a sequence of ribs and cavities, which reproduce, in negative, a fingerprint pattern and, consequently, they are suitable for providing the recessed and/or relief pattern 33.

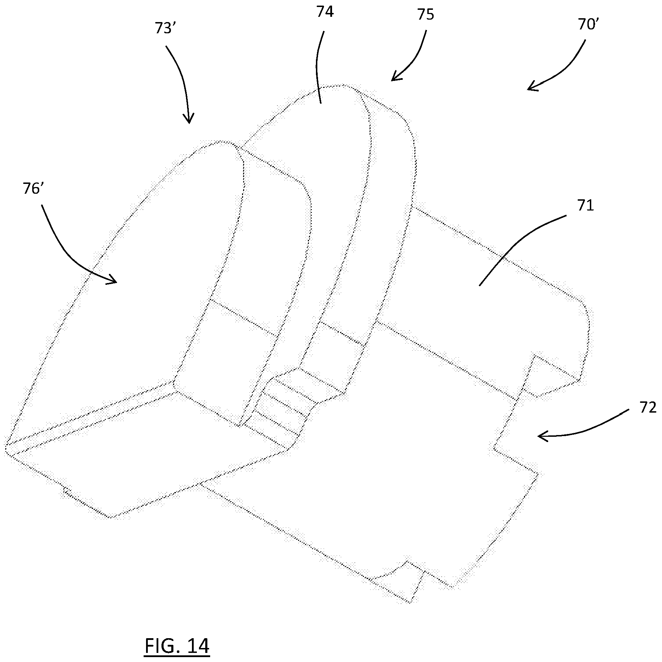

Fig. 14 shows a second casting die, 70', in which the head portion 73' is higher and the casting surface 76' is smooth. Therefore, the die 70' is suitable for making the inner layer 31 of the coating 30.

In the assembled casting configuration, the item 10, with the positioning members 60 assembled therewith, is housed and locked within the casting shell 100 with the sensorized surface 12 turned to face the casting surface 76 of the die 70. The sensorized surface 12 provided with tactile sensors 21, together with the casting 76 of the die 70 and the inner walls of the casting shell 100, determine the shape and dimensions of the casting chamber, which is in communication with the sprue 125, 135.

The shown embodiment of the casting device of the invention is particularly simple and suitable for being composed manually in the assembled casting configuration. For this purpose, the means for reciprocal fastening of the various components of the casting shell 100 consist of simple bolts and the means for fastening the die 70 to the casting shell 100 consist of an elastic ring-shaped element, not shown, that can be housed in the grooves 117, 143, 72.

Manual assembling and disassembling of the device of the invention are particularly preferred because of its constructive simplicity deriving therefrom, when prototypes, samples or small series of a given model of artificial finger are to be produced. In this case, it is also advantageous to provide an integrated design of the item 10, positioning members 60, casting shell 11 and dies 70, 70', which consequently can all be made, even of the same material, by rapid prototyping processes.

Obviously, other embodiments can provide the automation of the assembling and disassembling movements of the casting device 50.

According to the method of the present invention, a coating of silicone material that reproduces a fingerprint on the surface of a sensorized end of a fingertip of an artificial finger is obtained using the above described casting device 50 in the following way.

In the steps preceding the casting, the item 10, in the present embodiment consisting of the fingertip of an artificial finger, is removably associated to a first positioning element 61, which facilitates the grip and manual handling of the fingertip, in consideration of its small dimensions. A tactile sensor 20 is then coupled to the item 10, preferably by gluing. The sensor 20 consists of an array of tactile sensors of MEMS type 21, which are applied to a terminal surface 12, which can be provided with sensors, of the item 10, while further control and power electronics 22, suitably mounted on the PCB components, is assembled in suitable cavities or housings of the item 10.

The proper casting process begins with the coupling of the second positioning element 62 to the item 10. As previously described, in the present embodiment, the coupling is obtained by hinge couping means 11 of the fingertip and a respective intermediate phalange of the artificial finger.

The group consisting of the sensorized item 10, 20 and positioning members 60 is then locked between the lateral elements 120, 130 of the casting shell. Then, also the lower element 110 and finally the rear element 140 are assembled with the previously made group. The elements of the casting shell in the assembled casting configuration are then reciprocally locked by fastening means such as screws and bolts.

Afterwards, the casting die 70' is coupled with the casting shell 100 by introducing it into the opening 111 of the lower element 110 and keeping it in place by the previously described elastic ring-like element.

Once the assembled casting configuration of the device 50 has been completed, the sensorized surface 12, 21 of the item 10, the casting surface 76' of the die 70' and portions of the inner walls of the casting shell 100 form a casting chamber, which is in communication with the outside via the sprues 125, 135.

The casting surface 76' and the inner walls of the casting shell can be previously coated, by painting or by other means, with suitable substances, which facilitate the removal of the material of the coating 30 without reacting chemically with the material.

The plastic material, preferably silicone, in liquid state, is then poured into the sprue, until the casting chamber is filled.

Once the silicone material has solidified, which can occur, depending on the silicone material being used, either by cooling or by introduction of energy, for example, in the form of UV rays, the casting device is disassembled by removing the components in the reverse order.

The first layer 31 of coating 30 remains adhering to the sensorized end of the item 10. Burrs, sprues or other residues of the casting process are eliminated from the first layer 31 of the coating 30 without removing the positioning members 60.

Afterwards, the group formed by the sensorized item 10, 20 and positioning members 60 is locked again in the casting shell 100 which is assembled again in the casting configuration in the same previously described way.

The casting shell is associated, in the previously described way, with the casting die 70, which includes a casting surface 76 suitable for obtaining the recessed and/or relief pattern 33 reproducing a fingerprint.

Thus, also the steps including casting, solidification, disassembling of the casting device 50 and removal of the casting residues are repeated.

Finally, the positioning members 60 are removed to obtain the sensorized fingertip provided with silicone coating, which reproduces the pattern of a fingerprint, as shown in Figs. 4 and 5.

Advantageously, the two casting steps are performed using different silicone materials, and in particular, for the first casting, which produces the inner layer 31, a softer material is used and for the second casting, which produces the outer layer 32, a harder material is used.

Embodiments of the above described method can also provide a coating 30 with only one layer and, consequently, the use of only one die 70 and with only one casting operation.

Similarly, more than two overlaying layers of silicone material can be provided, performing the same number of castings as the number of layers to obtain, and providing a corresponding number of different dies.

These and other changes can be applied to the method and device of the invention, without departing from the protective scope defined by the following claims.

## Claims

1. A method for applying a plastic coating to a sensorized item (10, 20) comprising the steps of:
a) - associating removable positioning members (60) to said item (10),
b) - assembling the group made of said item (10) and said positioning members (60) into a multi-part casting shell (100),
c) - assembling a die (70, 70') with said casting shell (100),
d) - casting a silicone material in a non solid state into said shell (100),
e) - solidification of said silicone material,
f) - disassembling said die and shell (100),
g) - removing any casting waste material,
wherein said sensorized item (10, 20), said die (70, 70') and said shell (100), in the assembled configuration, form a casting chamber, with a first wall of said casting chamber being formed by a surface provided with an array of sensors (21) of said item (10), and a second wall, substantially opposite to said first wall, being formed by a shaped surface provided with a recessed and/or relief pattern (33) of said die (70, 70').

2. A method according to claim 1 **characterized in that** said steps b) to g) are performed at least twice with different dies, which are adapted to define, thanks to a different sizing, a different height of the casting chamber so that at least two superimposed layers of said silicone coating are obtained.

3. A method according to the previous claim **characterized in that** said step d) is repeated with different silicone materials having increasing hardness.

4. A method according to any preceding claim, **characterized in that** said sensorized item (10, 20) is a fingertip of an artificial finger, which is provided with MEMS-based tactile sensor array (21), and a die (70, 70') for realizing an outer layer of said coating is so shaped that its pattern mimics a fingerprint.

5. A method according to the previous claim, **characterized in that** said fingertip, said casting shell (100) and said die (70, 70') are made by rapid prototyping.

6. A casting device for applying a plastic coating to a sensorized item (10, 20), comprising:
- a multi-part casting shell (100),
- positioning members (60) which can be removably associated to said sensorized item (10, 20) and adapted to help the positioning of said sensorized item (10, 20) in said casting shell (100), and
- at least a casting die (70, 70'),
wherein said sensorized item (10, 20), said die (70, 70') and said shell (100), in the assembled configuration, form a casting chamber, with a first wall of said casting chamber being formed by a surface provided with an array of sensors (21) of said item (10), and a second wall of said casting chamber, substantially opposite to said first wall, being formed by a surface provided with a recessed and/or relief pattern (33) of said die (70, 70').

7. A device according to claim 6, **characterized in that** it includes a plurality of said dies (70, 70') which can be associated to said casting shell (100), each of said dies being shaped and sized so that it realizes a casting chamber adapted to realize a specific layer of a plurality of layers forming said plastic coating.

8. A device according to claim 6 or 7, **characterized in that** said multi-part shell (100) includes:
- a lower element (110) for housing said die (70, 70'),
- a pair of lateral elements shaped (120, 130) in a complementary way for housing, in the assembled configuration, said rigid sensorized item (10, 20),
- a rear element (140) provided with a housing portion of said sensorized item (10, 20).

9. A device according to claim 6 or the subsequent ones, **characterized in that** said positioning members (60) include a holding element (61) associated to said rigid sensorized item (10, 20) for easy handling, and a referencing bar (62), which can be associated through pin holes (11) of said sensorized item (10, 20), said bar (62) being attached externally to said casting shell (100).

## Patentansprüche

1. Verfahren zum Einführen von einer Kunststoffbeschichtung auf einem sensorisierten Artikel (10, 20), mit den folgenden Schritten:
a) - Assoziieren von abnehmbaren Positionierungselementen (60) an den Artikel (10),
b) - Zusammensetzen der aus dem Artikel (10) und der Positionierelementen (60) gebildet Gruppe in einer mehrteiligen Gießform (100),
c) - Zusammensetzen einer Matrize (70, 70') mit der Gießform (100),
d) - Gießen eines Silikon-Materials in einem nicht festen Zustand in die Gießform (100),
e) - Erstarren des Silikon-Materials,
f) - Auseinandernehmen der Matrize und der Gießform (100),
g) - Entfernen jedes Gieß-Abfallmaterials,
wobei der sensorisierte Artikel (10, 20), die Matrize (70, 70') und die Gießform (100), wen in der zusammengebauten Konfiguration sind, eine Gießkammer bilden, wobei eine ersten Wand der Gießkammer durch eine Oberfläche mit einer Anordnung von Sensoren (21) des Artikels (10) gebildet ist, und eine zweite Wand, die im wesentlichen entgegengesetzt zu dem ersten Wand sich befindet, durch eine mit einem vertieften und/oder abgebaut Muster (33) geformte Oberfläche der Matrize (70, 70') gebildet ist.

2. Verfahren nach Anspruch 1, **dadurch gekennzeichnet, dass** die Schritte b) bis g) mindestens zweimal mit unterschiedlichen Matrizen durchgeführt werden, wobei di Matrizen um eine unterschiedliche Höhe der Gießkammer durch eine unterschiedlichen Dimensionierung zu definieren angepasst sind, so dass zumindest zwei überlagerte Schichten der Silikonbeschichtung erhalten werden.

3. Verfahren nach dem vorigen Anspruch, **dadurch gekennzeichnet, dass** der Schritt d) mit unterschiedlichen Silikonmaterialien mit zunehmender Härte wiederholt.

4. Verfahren nach einem der vorhergehenden Ansprüche, **dadurch gekennzeichnet, dass** der sensorisierte Artikel (10, 20) ein Fingerspitze eines künstlichen Fingers ist, das mit MEMS-basierte taktile Sensorarray (21) vorgesehen ist, und wobei eine Matrize (70, 70') für die Realisierung eine Außenschicht der Beschichtung so geformt ist, dass sein Muster einen Fingerabdruck imitiert.

5. Verfahren nach dem vorigen Anspruch, **dadurch gekennzeichnet, dass** die Fingerspitze, die Gießform (100) und die Matrize (70, 70') durch schneller Prototypenbau hergestellt sind.

6. Eine Gießvorrichtung zum Einführen von einer Kunststoffbeschichtung auf einem sensorisierten Artikel (10, 20), umfassend:
- eine mehrteiligen Gießform (100),
- Positionierungselemente (60), die entfernbar zu dem sensorisierten Elemente (10, 20) verbunden sein kann, und die um die Positionierung des sensorisierten Artikels (10, 20) in der Gießform (100) zu helfen angepasst sind, und
- mindestens eine Gießform (70, 70'),
wobei der sensorisierte Artikel (10, 20), die Matrize (70, 70') und die Gießform (100), wen in der zusammengebauten Konfiguration sind, eine Gießkammer bilden, wobei eine ersten Wand der Gießkammer durch eine Oberfläche mit einer Anordnung von Sensoren (21) des Artikels (10) gebildet ist, und eine zweite Wand der Gießkammer, die im wesentlichen entgegengesetzt zu dem ersten Wand sich befindet, durch eine mit einem vertieften und/oder abgebaut Muster (33) geformte Oberfläche der Matrize (70, 70') gebildet ist.

7. Vorrichtung nach Anspruch 6, **dadurch gekennzeichnet, dass** die eine Mehrzahl von Matrizen (70, 70') enthält, die mit der Gießform (100) verbunden sein können, wobei jede der Matrizen so geformt und bemessen ist, dass die eine Gießkammer realisiert, die eine spezifische Schicht aus einer Vielzahl von Schichten bildet, die die Kunststoffbeschichtung bilden.

8. Vorrichtung nach Anspruch 6 oder 7, **dadurch gekennzeichnet, dass** die mehrteiligen Gießform (100) die folgendes aufweist:
- ein unteres Element (110) zum Aufnehmen der Matrize (70, 70'),
- ein Paar von in komplementärer Weise geformten Seitenelementen (120, 130), die zum Gehäuse des starren sensorisierten Artikels (10, 20), in der zusammengebauten Konfiguration, geeignet sind,
- ein hinteres Element (140), das mit einem Gehäuseabschnitt des genannten sensorisierten Artikels (10, 20) vorgesehen ist.

9. Vorrichtung nach Anspruch 6 oder den nachfolgenden Anspruche, **dadurch gekennzeichnet, dass** die Positionierungselementen (60) ein Halteelement (61) aufweisen, das für eine einfache Handhabung zu den starren sensorisierten Artikel (10, 20) zugeordnet ist, und eine Referenzierend Leiste (62), die durch Stiftlöcher des sensorisierten Artikels in Verbindung gebracht werden kann (11), aufweist, wobei die Leiste (62) von außen an der Gießform (100) angebracht ist.

## Revendications

1. Procédé pour appliquer un revêtement en matière plastique à un élément muni de capteurs (10, 20), comprenant les étapes de:
a. - l'union des éléments de positionnement amovibles (60) audit élément (10),
b. - l'assemblage du groupe constitué par ledit élément (10) et lesdits éléments de positionnement (60) dans une coquille de coulée en plusieurs parties (100),
c. - l'assemblage d'une matrice (70, 70') avec ladite coquille de coulée (100),
d. - la coulée d'un matériau de silicone dans un état non solide dans ladite coquille (100),
e. - la solidification dudit matériau de silicone,
f. - - le démontage de ladite matrice et de la coquille (100),
g. - l'élimination de tout matériau de déchets de coulée,
dans lequel lesdites élément muni de capteurs (10, 20), ladite matrice (70, 70') et ladite coquille (100), dans la configuration assemblée, forment une chambre de coulée, avec une première paroi de ladite chambre de coulée étant formée par une surface munie d'un réseau de capteurs (21) dudit article (10), et une deuxième paroi, substantiellement opposée à ladite première paroi, étant formée par une surface profilée pourvue d'un motif en retrait et/ou en relief (33) de ladite matrice (70, 70').

2. Procédé selon la revendication 1, **caractérisé en ce que** lesdites étapes b) à g) sont effectuées au moins deux fois avec différentes matrices, qui sont adaptés pour définir une hauteur différente de la chambre de coulée, grâce à un dimensionnement différent, de façon à obtenir au moins deux couches superposées dudit revêtement de silicone.

3. Procédé selon la revendication précédente, **caractérisé en ce que** ladite étape d) est répétée avec différents matériaux de silicone ayant une dureté croissante.

4. Procédé selon l'une quelconque des revendications précédentes, **caractérisé en ce que** ledit élément muni de capteurs (10, 20) est un bout de doigt d'un doigt artificiel, qui est muni d'ensemble de capteur tactile à base de MEMS (21), et une matrice (70, 70') pour réaliser une couche extérieure dudit revêtement a une forme telle que son forme imite une empreinte digitale.

5. Procédé selon la revendication précédente, **caractérisé en ce que** ledit bout de doigt, ladite coquille de coulée (100) et ladite matrice (70, 70') sont réalisés par un prototypage rapide.

6. Dispositif de moulage pour appliquer un revêtement en matière plastique à un élément muni de capteurs (10, 20), comprenant:
- une coquille de coulée en plusieurs parties (100),
- des éléments de positionnement (60) qui peuvent être associés de façon amovible audit élément muni de capteurs (10, 20) et ils sont adaptés pour aider au positionnement dudit élément muni de capteurs (10, 20) dans ladite coquille de coulée (100), et
- au moins une matrice de coulée (70, 70'),
dans lequel ledit élément muni de capteurs (10, 20), ladite matrice (70, 70') et ladite coquille (100), dans la configuration assemblée, forment une chambre de coulée, avec une première paroi de ladite chambre de coulée étant formée par une surface munie d'un réseau de capteurs (21) dudit article (10), et une deuxième paroi dédite chambre de coulée, substantiellement opposée à ladite première paroi, étant formée par une surface pourvue d'un motif en retrait et/ou en relief (33) de ladite matrice (70, 70').

7. Dispositif selon la revendication 6, **caractérisé en ce que** il comprend une pluralité desdites matrices (70, 70') qui peuvent être associés à ladite coquille de coulée (100), chacune desdites matrices étant formé et dimensionné de sorte qu'il forme une chambre de coulée adaptée pour réaliser une couche spécifique d'une pluralité de couches formant ledit revêtement en matière plastique.

8. Dispositif selon les revendications 6 ou 7, **caractérisé en ce que** ladite coquille en plusieurs parties (100) comprend:
- un élément inférieur (110) pour loger ladite matrice (70, 70'),
- une paire d'éléments latéraux (120, 130) ayant une forme complémentaire pour le logement, dans la configuration assemblée, dudit élément muni de capteurs (10, 20),
- un élément arrière (140) pourvue d'une partie de boîtier dudit élément muni de capteurs (10, 20).

9. Dispositif selon la revendication 6 ou les suivantes, **caractérisé en ce que** lesdits éléments de positionnement (60) comprennent un élément de maintien (61) associé audit élément muni de capteurs (10, 20) rigide pour une manipulation facile, et il comprend une barre de référence (62), qui peut être associé par l'intermédiaire des petits trous (11) dudit élément muni de capteurs (10, 20), ladite barre (62) étant fixé extérieurement à ladite coquille de coulée (100).
